# EUROPEAN PATENT APPLICATION

(11) **EP 3 756 693 A2**
(43) Date of publication of application: **30.12.2020**
(21) Application number: 20181407.6
(22) Date of filing: 22.06.2020
(51) Int. Cl.: A61L 2/20, A61L 2/18, B65B 55/10, B65D 5/74

(54) **A METHOD FOR REMOVING EXCESS OF A STERILIZATION AGENT FROM A WEB OF CARTON-BASED PACKAGING MATERIAL AND A SYSTEM THEREOF**

(30) Priority: 28.06.2019 EP 19183383
(71) Applicant: Tetra Laval Holdings & Finance S.A., 1009 Pully (CH)
(72) Inventor: SAEIDIHAGHI, Arash, 24735 Södra Sandby (SE)
(74) Representative: Tetra Pak - Patent Attorneys SE

(57) **Abstract**

An apparatus (312) for removing excess of a sterilization agent (308) from a web (102) of carton-based packaging material is presented. The web (102) comprises a number of consecutively arranged sections (200a-200f) arranged to be formed into packages (112), wherein each section of the web (102) comprises an opening device (202a-f) attached thereto, wherein at least a subset of the sections (200a-f) comprises a positioning element (204a-f). The apparatus (312) comprises a positioning element reader (314) arranged to determine a position of the positioning element (204a-f) comprised in or on the web (102), a control unit (316) configured to estimate a position of the opening device (202a-f) based on the position of the positioning element (204a-d), and an air pulse device (318) arranged to provide an air pulse (320a-b), wherein the control unit (316) is further configured to adjust the air pulse device (318) based on the position of the opening device, and the air pulse (320a-b) is provided onto an opening device area (210a-f) of the web (102) such that sterilization agent (308) is removed from the opening device (202a-f).

## Description

### Technical Field

The invention generally relates to the field of packaging technology, such as food packaging technology. More particularly, it is presented a method and apparatus for removing excess of a sterilization agent from a web of a packaging material.

### Background Art

Today it is commonly known to use roll-fed packaging machines for different types of food products, e.g. milk. The roll-fed packaging machines, also referred to as filling machines, have several advantages. One is that a continuous production of packages makes it possible to achieve higher speed compared to blanks-fed packaging systems. Another advantages is that by continuously filling a tube of packaging material and forming packages in a lower end of the tube, a risk that unwanted microorganisms enter the packages can be lowered.

For ensuring that the packaging material is free from unwanted microorganisms, the packaging material can be fed to a sterilization bath. The sterilization bath contains a sterilization agent only or a mixture of a sterilization agent and a wetting agent. In this text, to ease the understanding, the liquid in the bath will be named as sterilization agent whether it is together with a wetting agent as a mixture or not. A drawback with the sterilization bath is that the sterilization agent used in the sterilization bath need to be removed to provide for that this, or these, does not end up in the product. One way of removing the sterilization agent is to dry the packaging material be using heat-sterilized air.

Even though technologies today exist for making sure that the sterilization agent is adequately removed, there is still a need for improved technologies in order to provide for that this can be achieved more efficiently, for instance, in terms of energy usage.

### Summary

It is an object of the invention to at least partly overcome one or more of the above-identified limitations of the prior art. In particular, it is an object to provide an apparatus for removing excess of a sterilization agent from packaging material by using air pulses.

According to a first aspect it is provided an apparatus for removing excess of a sterilization agent from a web of carton-based packaging material. The web comprises a number of consecutively arranged sections arranged to be formed into packages, wherein each section of the web comprises an opening device attached thereto, wherein at least a subset of the sections comprises a positioning element. The apparatus comprising
a positioning element reader arranged to determine a position of the positioning element comprised in or on the web ,
a control unit configured to estimate a position of the opening device based on the position of the positioning element, and
an air pulse device arranged to provide an air pulse,
wherein the control unit is further configured to adjust the air pulse device based on the position of the opening device, and the air pulse is provided onto an opening device area of the web such that excess of the sterilization agent is removed from the opening device.

The opening device may be an injection molded opening device perforating the web.

The air pulse device may be arranged to provide air pulses on both sides of the web.

The air pulse device may comprise an air pulse pump arranged for building up an air pulse pressure between consecutive air pulses, and an air pulse valve configured to release the air pulse pressure such that the air pulse is formed.

The air pulse valve may be a radial valve or a solenoid valve.

The air pulse pressure when opening the air pulse valve may be 1.5 to 5.0 bar, preferably 2.0 bar.

The sterilization agent may comprise hydrogen peroxide.

The air pulse device may be configured to provide 3 to 10 air pulses per second.

The positioning element may be a magnetic mark integrated in the web.

According to a second aspect it is provided a packaging system. The packaging system comprises
a packaging material receiver arranged to receive a web of packaging material, an opening device provider arranged to provide opening devices to the web, a sterilization bath arranged to provide a sterilization agent onto the web,
an apparatus according to the first aspect (any one of the claims 1 to 8), wherein the apparatus is arranged to remove excess of the sterilization agent from opening device areas of the web,
a sterilization chamber in which the web is sterilized, and an aseptic chamber in which the web is formed into packages.

The opening device provider may be an injection molding apparatus arranged to provide injection molded opening devices perforating the web.

According to a third aspect it is provided a method for removing excess of a sterilization agent from a web of carton-based packaging material. The web comprises a number of consecutively arranged sections arranged to be formed into packages, wherein each section of the web comprises an opening device attached thereto, wherein at least a subset of the sections comprises a positioning element. The method comprising
determining a position of the positioning element comprised in or on the web,
estimating a position of the opening device based on the positioning element,
adjusting an air pulse device based on the position of the opening device, and
providing an air pulse onto an opening device area of the web such that excess of the sterilization agent is removed from the opening device.

The opening device may be an injection molded opening device perforating the web.

The method may further comprise
providing the sterilization agent onto the web by feeding this through a sterilization bath before the step of determining the position of the positioning element.

The method may further comprise
building up an air pulse pressure by using an air pulse pressure pump, and
wherein the step of providing the air pulse pressure is performed by releasing an air pulse pressure valve such that the air pulse is formed.

Still other objectives, features, aspects and advantages of the invention will appear from the following detailed description as well as from the drawings.

### Brief Description of the Drawings

Embodiments of the invention will now be described, by way of example, with reference to the accompanying schematic drawings, in which
Fig. 1 is a general illustration of a roll-fed packaging material.
Fig. 2a-d illustrates different examples of how a web of a packaging material can be arranged.
Fig. 3 schematically illustrates a packaging system.
Fig. 4 is a flow chart illustrating a method for removing excess of a sterilization agent from a web of carton-based packaging material.

### Detailed Description

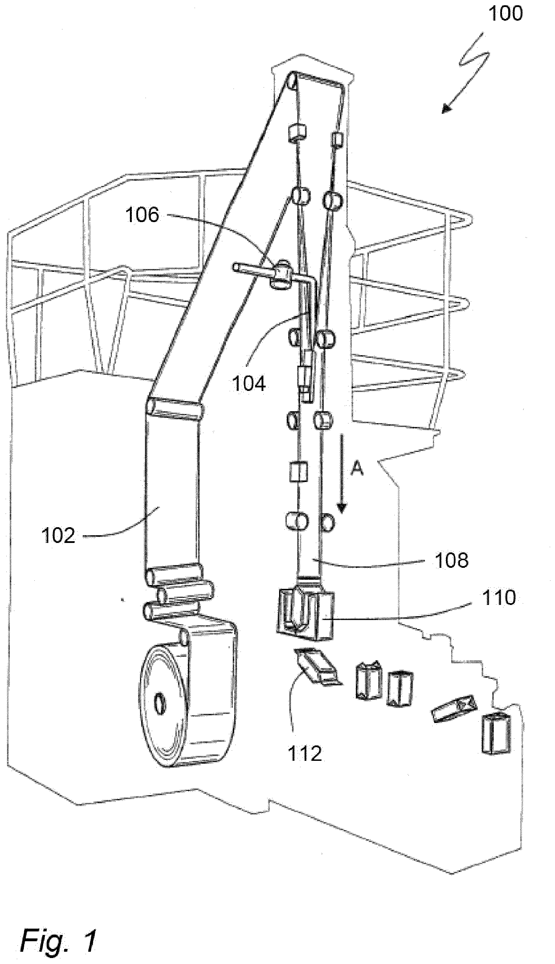

With reference to Fig. 1, a packaging machine 100, sometimes also referred to as a filling machine, is generally illustrated by way of example. The packaging machine 100 is a roll-fed packaging machine used for packaging liquid food products in carton-based packages. Already in the 1940s this type of packaging machines was introduced by Tetra Pak, and it is today a well-known approach for packaging milk and other liquid food products in a safe and cost-efficient manner. The general approach can also be used for non-liquid food products, such as potato crisps.

Today, packaging material is often printed and prepared in packaging material production centers, also referred to as converting factories, and is shipped to a site where the packaging machine 100 is placed, e.g. a dairy. Usually the packaging material is loaded onto a reel before being transported. After arriving at the site, the reel is placed in the packaging machine as illustrated in Fig. 1.

During production a web 102 of packaging material can be fed from the reel through the packaging machine in a web feeding direction A. Even though not illustrated in Fig. 1, the packaging material may pass through a sterilization device, e.g. a hydrogen peroxide bath or an LVEB (Low-Voltage Electron Beam) station, for ensuring that the web 102 is free from unwanted microorganisms. Before providing the food product, a tube can be formed from the web 102 by forming a longitudinal sealing. The food product can be fed into the tube via a pipe 104 and a valve 106 may be used for regulating a flow through the pipe 104. A lower end 108 of the tube can be fed into a folding device 110 in which a transversal sealing is made, the tube is folded according to folding lines, also referred to as weakening lines, and cut off such that packages 112 can be formed. Even though the folding device 110 is illustrated as one single device, the folding device 110 may comprise a number of different devices.

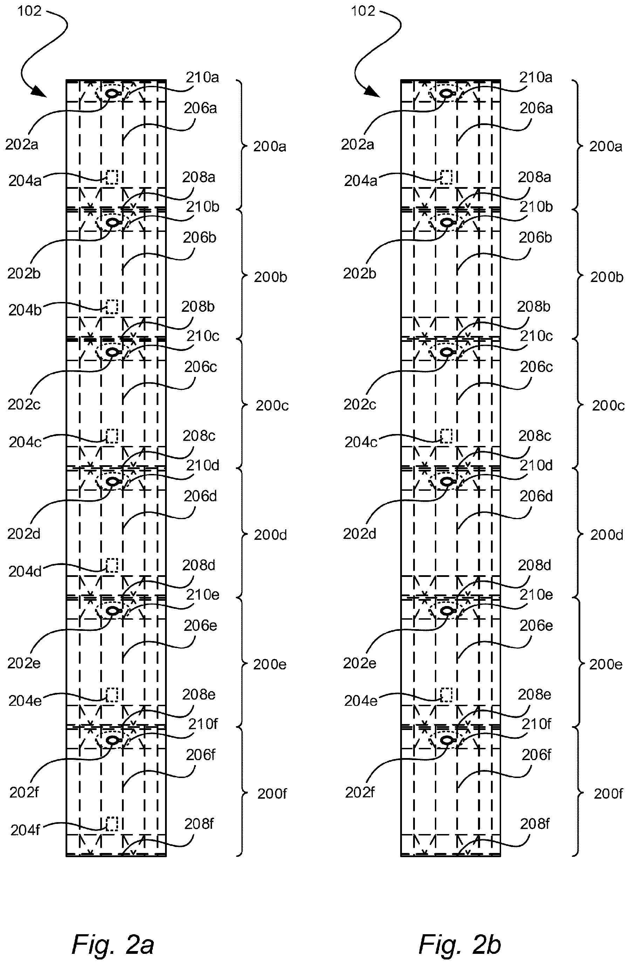

With reference to Fig. 2a-d, a web 102 of a carton-based packaging material is illustrated by way of example. The web 102 may comprise a number of consecutively arranged sections 200a-f. Each of the number of consecutively arranged sections 200a-f may comprise an opening device 202a-f attached thereto. At least a subset of the number of consecutively arranged sections 200a-f may comprise a positioning element 204a-f.

According to Fig. 2a and 2b, each of the number of the consecutively arranged sections 200a-f may comprise a number of folding lines 206a-f. The number of folding lines 206a-f may be based on what type of package 112 the web 102 is to be folded into. Further, each of the number of consecutively arranged sections 200a-f may comprise a transversal sealing and a longitudinal sealing. The transversal sealing may be arranged to form a top and a bottom of a folded package, while the longitudinal sealing may be arranged to seal the folded package along the side.

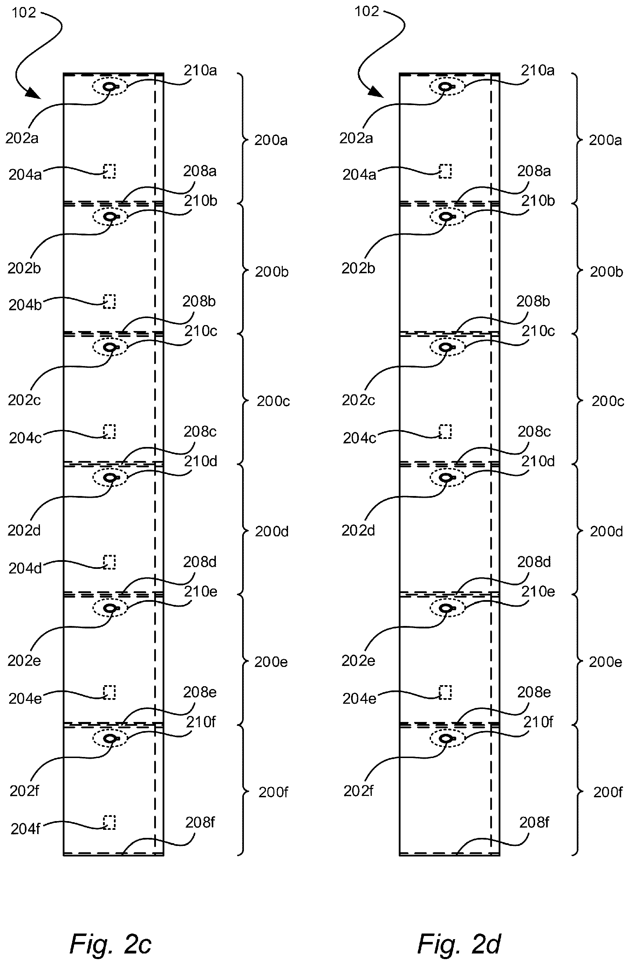

As illustrated in Fig. 2c and 2d by way of example, the number of the consecutively arranged sections 200a-f may comprise only the transversal sealing and the longitudinal sealing, but not the number of folding lines 206a-f. With this set folding lines the packages 112 being formed will have a bag-like appearance, instead of the brick-shaped appearance of the packages 112 formed by the web 102 illustrated in Fig. 2a and 2b.

An opening device 202a-f may be arranged to provide an easy opening which can be used after the web 102 has been formed to the packages 112. The opening device 202a-f may be arranged to provide a predetermined way for opening the packages 112. The opening device 202a-f may be an injection molded opening device, wherein the injection molded opening device may be perforating the web 102. An advantage with this type of openings, sometimes referred to as micro-injected molded openings, is that an amount of plastics being used may be reduced compared to spout and screw cap based opening devices.

An positioning element 204a-f may be comprised in or on the web 102. For instance, the positioning element 204a-f, which may be a magnetic mark, may be integrated in the packaging material such that a position of this is remained during production, herein referred to as an information carrier. Alternatively, by way of example, the positioning element 204a-f may be provided on the packaging material as an optical reference mark, e.g. a printed mark provided thereon.

In this context the positioning element 204a-f may be used for providing a reference position in the web 102 to the packaging machine. Put differently, the positioning element 204a-f can be used for providing information about the position of the web to the packaging machine. The positioning element 204a-f may be a mark and/or code that can be read by a photocell, a camera or a tape reader. Optionally, as described above, the positioning element 204a-f may be the information carrier. The positioning element 204a-f is not limited to any of these examples but can be any reference position providing information about the position of the web. According to one example, the opening device 202a-f itself can be arranged to work as a visual reference point as well. As illustrated in Fig. 2a and 2c, each of the number of consecutively arranged sections 200a-f may comprise the positioning element 204a-f. Having this set-up, a position of the opening device 202a-f may be determined based on a position of the positioning element 204a-f placed in the same section. The positioning element 204a-f and the opening device 202a-f do not have to be placed in the same section however. As illustrated in Fig. 2b and 2d, the positioning elements 204a-f may only be provided in a subset of the sections 200a-f. An accuracy with which the positions of the opening devices 202a-f are determined may be lower compared to the examples illustrated in Fig. 2a and 2c, but since less positioning elements 204a-f are being used a production cost of the packaging material may be lowered. Put differently, one of the positioning element 204a-f may be used for estimating the positions of a plurality of the opening devices 202a-f.

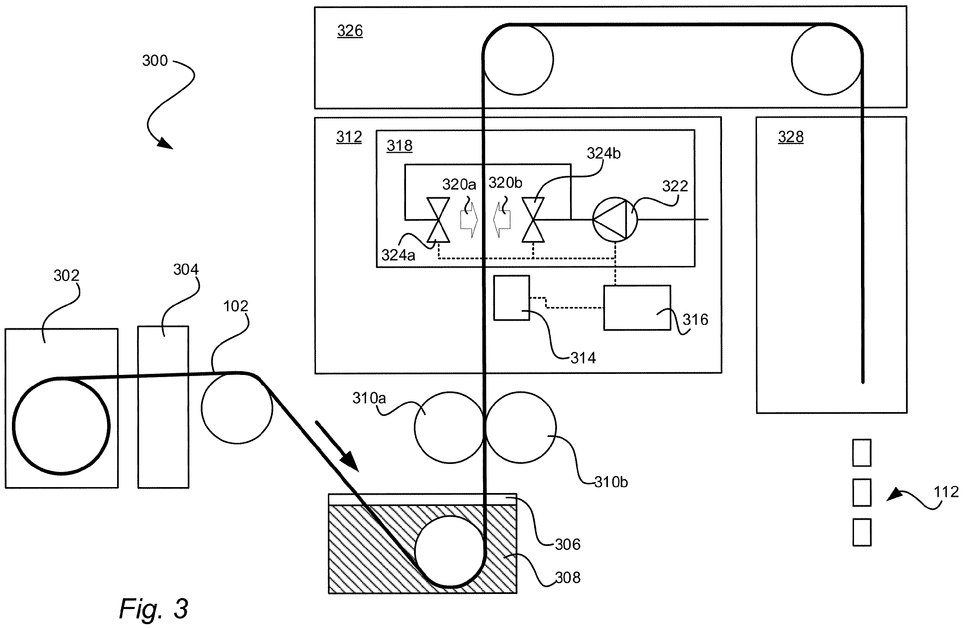

With reference to Fig. 3, a packaging system 300 is illustrated by way of example. The packaging system 300 may comprise a packaging material receiver 302, an opening device provider 304, a sterilization bath 306, an apparatus 312, a sterilization chamber 326 and an aseptic chamber 328.

The packaging system 300 may be arranged to form the packages 112 from the web 102 of carton-based packaging material. The web 102 may be fed into the packaging system 300 via the packaging material receiver 302. Thereafter, the web 102 may be fed to the opening device provider 304. The opening device provider 304 may be arranged to provide the opening device 202a-f to the web 102, for instance by using an injection molding apparatus. This may be arranged to provide injection molded opening devices perforating the web 102. This may be made possible by having perforations in a carton layer of the web 102, i.e. weakened sections of the web such that melted plastics can pushed through.

In order to sterilize the web 102, the web 102 may be fed into the sterilization bath 306. The sterilization bath 306 may be arranged to provide a sterilization agent 308 onto the web 102. The sterilization agent 308 may be arranged to ensure that germs and other unwanted microorganisms are removed from the packaging material. According to one example, the sterilization agent 308 may comprise hydrogen peroxide. To remove excessive sterilization agent 308 and/or to provide for that the sterilization agent is spread over the web, rollers 310a,b may be used.

Having the opening devices 202a-f provided on the web 102 may result in that the sterilization agent 308 is more difficult to remove. Put differently, the opening devices 202a-f on the web 102 result in that protrusions are provided on the web 102, which in turn result in that excessive sterilization agent 308 may remain in or close to the opening devices 202a-f.

Here the "excessive" may mean any amount of sterilization agent exists on surfaces on or near the opening devices 202a-f excessing thickness of a sterilization agent layer as predefined. This predefined thickness may be a value of thickness that is provided on the web by the rollers 310a,b. For example, it may be a value in between 1-5 mm, more preferably 1-2 mm. This excessive amount may be removed by blowing air pulse with predefined pressure as explained later and to a predefined position that is controlled by the positioning element 204a-f and the positioning element reader 314.

Here the "remove" may mean relocating the excessive amount of the sterilization agent from the opening device 210a-f to somewhere else. This somewhere may be the web 102 around the opening device 210a-f. The excessive sterilization agent may be removed in the sterilization chamber 326 as explained later.

To remove excess of the sterilization agent 308 held in the opening devices 202a-f, the apparatus 312 may comprise a positioning element reader 314, a control unit 316 and an air pulse device 318. The positioning element reader 314 may be arranged to determine the position of the positioning element 204a-f, wherein the positioning element 204a-f may be comprised in or on the web 102, as discussed above.

The control unit 316 may be arranged to estimate the position of the opening device 202a-f based on the position of the positioning element 204a-f. How the position of the positioning element 202a-f is related to the position of the opening device 202a-f can be known based on information from the packaging material production center.

The air pulse device 318 may be arranged to provide an air pulse 320a-b such that excess of the sterilization agent is removed. The air pulse 320a-b may be provided towards the web 102. The air pulse device 318 may be arranged to provide 3 to 10 air pulses 320a-b per second. According to the example illustrated in Fig. 3, the air pulse device 318 may be arranged to provide air pulses 320a-b on both sides of the web 102. Alternatively, the air pulse device 318 may be arranged to only provide the air pulses 320a-b on one side of the web 102. Upon that may be the case, the air pulse 320a-b may preferably be provided on an inside of the web 102 since that may be the side with which the milk or the other liquid food products is in contact.

The control unit 316 may be arranged to adjust the air pulse device 318 based on the position of the opening device 202a-b, which as described above may be determined based on the position of the positioning element 202a-f. The air pulse 320a-b may be provided onto an opening device area 210a-f of the web 102, in which the opening device is placed. The opening device area 210a-f may be larger than an area of the opening device 202a-f such that the opening device 202a-f and a surrounding area is covered.

The air pulse device 318 may comprise an air pulse pressure pump 322. The air pulse pressure pump 322 may be arranged to build up an air pulse pressure between consecutive air pulses 320a-b. The air pulse device 318 may comprise an air pulse pressure valve 324a-b. The air pulse pressure valve 324a-b may be arranged to release the air pulse pressure such that the air pulses 320a-b are formed. The length of the air pulses 320a-b may be modified due to the length of the opening of the air pulse pressure valves 324a-b. The air pulse pressure valve 324a-b may be a radial valve or a solenoid valve. The air pulse 320a-b may be a high pressurized air pulse. Upon the air pulse pressure valve 324a-b is opened, the air pulse pressure may be 1.5 to 5.0 bar, preferably 2.0±0.1 bar. The control unit 316 may be arranged to control the air pulse pressure pump 322 and the air pulse pressure valves 324a-b. More particularly, the control unit 316 may be arranged to open the air pulse pressure valves 324a-b when the opening device 202a-f pass the air pulse device 318.

By using air pulses instead of continuously providing pressurized air, high pressurized air may be generated. Hence, instead of continuously providing an air flow, air pulses are used in areas, e.g. the opening device areas 210a-f, where excessive sterilization agent is likely to be found.

Using air pulses instead of the air flow is also beneficial from an energy efficiency perspective. By identifying areas of particular relevance, e.g. the opening device areas 210a-f, and providing air pulses in these areas, less energy may be needed compared to continuously providing the air flow. Also from a sterilization performance perspective, the air pulses may be prove beneficial. By only removing excess of the sterilization agent in the opening device areas 210a-f, other areas of the web 102 may be left untouched with the effect that the sterilization in these other areas may be improved.

When the web 102 has passed the apparatus 312, the web 102 may be fed to a sterilization chamber 326. The sterilization chamber 326 may be arranged to provide for that the web 102 is sterilized. To do so, hot air may be supplied on the web 102 and the opening device 210a-f to evaporate the sterilization agent existing on the web 102 and the opening device 210a-f. Thereafter, the web 102 may be fed to the aseptic chamber 328 in which the web 102 is formed into the packages.

By using the present system, as described above, less energy may be needed because of that air pulses are used instead of the continuous air flow. By using air pulses instead of continuous air flows, the total air flow amount can be much lower since the air only is needed for small areas where the injection molded openings are located. This is a more efficient solution for removing the excess of the sterilization agent compared to using continuous air flows when injection molded opening are on the packages.

Another advantage obtained, since the air only is directed to small parts of the web, is that this system does not interfere with the sterilization of the packaging material. Further, since the air only is directed to small parts of the web, the air will not warm up the packaging material and weaken the efficacy of the sterilization.

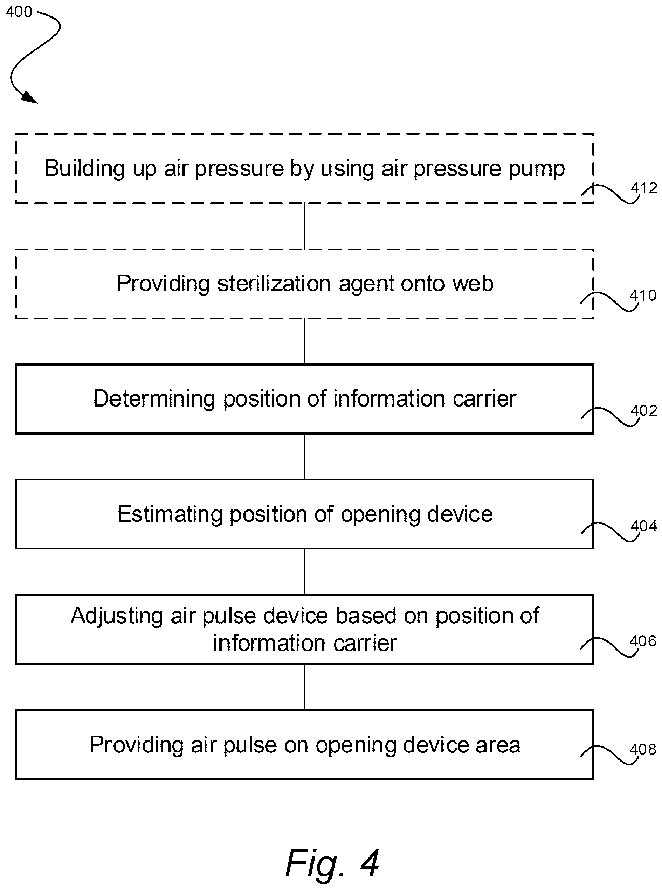

In Fig. 4 it is presented a flowchart illustrating a method 400 for removing excess of a sterilization agent 308 from a web 102 of carton-based packaging material by way of example. The web 102 may comprise a number of consecutively arranged sections 200a-f, wherein the consecutively arranged sections 200a-f may be arranged to be formed into packages 112. Each section of the web 102 may comprise an opening device 202a-f which may be attached thereto, and wherein at least a subset of the sections 200a-f may comprise a positioning element 204a-f.

In a first step 402, a position of the positioning element 204a-f can be determined. The positioning element 204a-f may be comprised in or on the web 102. In a second step 404, a position of the opening device 202a-f can be estimated based on the position of the positioning element 204a-f. In a third step 406, an air pulse device 318 can be adjusted based on the position of the opening device 202a-f. The opening device 202a-f can be an injection molded opening device which may be perforating the web 102. In a fourth step 408, an air pulse 320a-b can be provided onto an opening device area 210a-f of the web 102 such that excess of the sterilization agent 308 can be removed from the opening device 202a-f.

Optionally, in a fifth step 410, the sterilization agent 308 can be provided onto the web 102 by feeding the web 102 through a sterilization bath 306 before the step of determining 402 the position of the positioning element 204a-f.

Optionally, in a sixth step 412, an air pulse pressure can be built up by using an air pulse pressure pump 322, wherein the step of providing 408 the air pulse pressure can be performed by releasing an air pressure valve 324a-b such that the air pulse 320a-b can be formed.

Even if described in specific order it is to be understood that the steps do not have to be performed in this order.

From the description above follows that, although various embodiments of the invention have been described and shown, the invention is not restricted thereto, but may also be embodied in other ways within the scope of the subject-matter defined in the following claims.

## Claims

1. An apparatus (312) for removing excess of a sterilization agent (308) from a web (102) of carton-based packaging material, wherein the web (102) comprises a number of consecutively arranged sections (200a-200f) arranged to be formed into packages (112), wherein each section of the web (102) comprises an opening device (202a-f) attached thereto, wherein at least a subset of the sections (200a-f) comprises a positioning element (204a-f), said apparatus comprising
a positioning element reader (314) arranged to determine a position of the positioning element (204a-f) comprised in or on the web (102),
a control unit (316) configured to estimate a position of the opening device (202a-f) based on the position of the positioning element (204a-d), and
an air pulse device (318) arranged to provide an air pulse (320a-b),
wherein the control unit (316) is further configured to adjust the air pulse device (318) based on the position of the opening device, and the air pulse (320a-b) is provided onto an opening device area (210a-f) of the web (102) such that excess of the sterilization agent (308) is removed from the opening device (202a-f).

2. The apparatus according to claim 1, wherein the opening device (202a-f) is an injection molded opening device perforating the web (102).

3. The apparatus according to any one of the preceding claims, wherein the air pulse device (318) is arranged to provide air pulses (320a-b) on both sides of the web (102).

4. The apparatus according to any one of the preceding claims, wherein the air pulse device (318) comprises an air pulse pump (322) arranged for building up an air pulse pressure between consecutive air pulses, and an air pulse valve (324a-b) configured to release the air pulse pressure such that the air pulse (320a-b) is formed.

5. The apparatus according to claim 4, wherein the air pulse valve (324a-b) is a radial valve or a solenoid valve.

6. The apparatus according to any one of the preceding claims, wherein the air pulse pressure when opening the air pulse valve (324a-b) is 1.5 to 5.0 bar, preferably 2.0 bar.

7. The apparatus according to any one of the preceding claims, wherein the sterilization agent (308) comprises hydrogen peroxide.

8. The apparatus according to any one of the preceding claims, wherein the air pulse device (318) is configured to provide 3 to 10 air pulses per second.

9. The apparatus according to any one of the preceding claims, wherein the positioning element (204a-f) is a magnetic mark integrated in the web (102).

10. A packaging system (300) comprising
a packaging material receiver (302) arranged to receive a web (102) of packaging material,
an opening device provider (304) arranged to provide opening devices to the web (102),
a sterilization bath (306) arranged to provide a sterilization agent (308) onto the web (102),
an apparatus (312) according to any one of the claims 1 to 8, wherein the apparatus is arranged to remove excess of the sterilization agent (308) from opening device areas (210a-f) of the web (102),
a sterilization chamber (326) in which the web (102) is sterilized, and
an aseptic chamber (328) in which the web (102) is formed into packages (112).

11. The packaging system (300) according to claim 10, wherein the opening device provider (304) is an injection molding apparatus arranged to provide injection molded opening devices perforating the web (102).

12. A method (400) for removing excess of a sterilization agent (308) from a web (102) of carton-based packaging material, wherein the web (102) comprises a number of consecutively arranged sections (200a-f) arranged to be formed into packages (112), wherein each section of the web (102) comprises an opening device (202a-f) attached thereto, wherein at least a subset of the sections (200a-f) comprises a positioning element (204a-f), said method comprising
determining (402) a position of the positioning element (204a-f) comprised in or on the web (102),
estimating (404) a position of the opening device (202a-f) based on the position of the positioning element (204a-f),
adjusting (406) an air pulse device (318) based on the position of the opening device (202a-f), and
providing (408) an air pulse (320a-b) onto an opening device area (210a-f) of the web (102) such that excess of the sterilization agent (308) is removed from the opening device (202a-f).

13. The method according to claim 12, wherein the opening device (202a-f) is an injection molded opening device perforating the web (102).

14. The method according to any one of the claims 12 to 13, further comprising providing (410) the sterilization agent (308) onto the web (102) by feeding this through a sterilization bath (306) before the step of determining (402) the position of the positioning element (204a-f).

15. The method according to any one of the claims 12 to 14, further comprising
building up (412) an air pulse pressure by using an air pulse pressure pump (322), and
wherein the step of providing (408) the air pulse pressure is performed by releasing an air pulse pressure valve (324a-b) such that the air pulse (320a-b) is formed.
